# EUROPEAN PATENT SPECIFICATION

(11) **EP 0 490 311 B1**
(45) Date of publication and mention of the grant of the patent: **20.03.1996**
(21) Application number: 91121070.6
(22) Date of filing: 09.12.1991
(51) Int. Cl.: C07H 17/08, A61K 31/70

(54) **Derivatives of 10,11,12,13-tetra-hydrodesmycosin, processes for preparation, and use thereof in obtaining pharmaceuticals**
Derivate von 10,11,12,13-tetra-hydrodesmycosin, Verfahren zu ihrer Herstellung, und ihre Verwendung zur Herstellung von Arzneimitteln
Dérivés de 10,11,12,13-tetra-hydrodesmycosin, procédé pour leur préparation, et leur utilisation dans la production des pharmaceutiques

(30) Priority: 14.12.1990 YU 2363/90
(43) Date of publication of application: 17.06.1992
(73) Proprietor: PLIVA FARMACEUTSKA, KEMIJSKA, PREHRAMBENA I KOZMETICKA INDUSTRIJA S P.O., YU-41001 Zagreb (YU)
(72) Inventor: Narandja, Amalija, 41000 Zagreb (YU); Djokic, Slobodan, 41000 Zagreb (YU)
(74) Representative: von Füner, Alexander, Dr.

(56) References cited:
- EP-A- 0 070 170
- EP-A- 0 287 082
- EP-A- 0 376 114
- EP-A- 0 410 433
- US-A- 4 056 616

## Description

The invention relates to derivatives of tylosin, namely to novel products from the series of macrolide antibiotics possessing antimicrobial activity. The invention relates especially to derivatives of 10,11,12,13-tetrahydro-desmycosin represented by the formula I 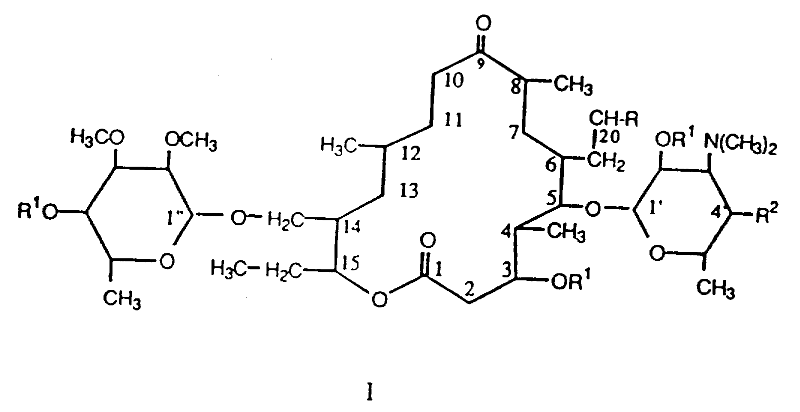 *wherein*
- *R*: *represents O when R¹ stands for H and R stands for H or I; or*
- *R*: *represents (OC₂H₅)₂ when R¹ stands for Si(CH₃)₃ and R stands for OH, OSO₂CH₃,I or H; or when R¹ stands for H and R stands for H or OH*.
and to their pharmaceutically acceptable addition salts, to processes for the preparation thereof, and to their use in obtaining pharmaceutical preparations.

Desmycosin is a 16-membered macrolide antibiotic obtained by hydrolysis of mycarose in position C-4' of the starting antibiotic tylosin (R.L. Hamill, Antibiotics and Chemotherapy **11** 328 (1961)).

10,11,12,13-tetrahydro-desmycosin was prepared by means of catalytical hydrogenation of desmycosin (Narandja *et al,* DD 272 304 A5 (1989)).

EP-A-287082 discloses derivatives of tylosin and 10,11,12,13-tetrahydro tylosin of the general formula 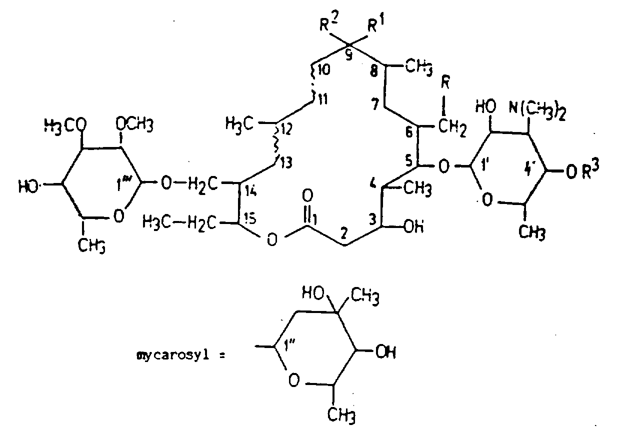 wherein R stands for CHO, CH₂OH, CH=NOH or CH(OCH₃)₂, R¹ stands for H, R stands for OH or R¹ + R stand for =O or =NOH, R³ stands for a mycarosyl group or a hydrogen atom, and m stands for a single or a double bond.

It has been known that 4'-deoxy-desmycosin was prepared by a reaction sequence, wherein the reactive groups which did not take part in the reaction were previously selectively protected, whereas they were deblocked upon the performed target reaction. Thus, A. Tanaka *et al* prepared 4'-deoxy-desmycosin *via* the following reaction sequence: acetylation of desmycosin in positions C-2' and 4', tetrahydro-furanisation in positions C-3 and 4", methanolysis of acetyl in C-2' and 4', selective sulfonation in C-4', substitution with iodine, deiodination by means of tin tributyl hydride, and hydrolysis of the tetrahydrofuranyl and acetyl protecting groups (J. Antibiot. **34** 1381(1981)).

It has been known that Fujivara *et al* also prepared 4'-deoxy-desmycosin *via* the reaction sequence comprising: selective acetylation of tylosin in position C-2', hydrolysis of mycarose, acetylation of the obtained desmycosin in position C-4", sulfonation in position C-4', substitution of the sulfonyl with iodine, deacetylation in position C-4", deiodination by means of tin tributyl hydride, and hydrolysis of the acetyl in C-2' (US 4,421,911 (1983)).

It has been known as well that A. Imai *et al* prepared 19-deformyl-4'-desmycosin upon deformylation of tylosin with a Wilkinson catalyst, silylation of the hydroxy in C-3,2' and 4", sulfonation in C-4', substitution of the sulfonyl with iodine, reduction of iodine with tin tributyl hydride, and hydrolysis of the silyl (J. Antibiot. **42** 903 (1989)).

According to the known prior art 4'-deoxy-10,11,12,13-tetrahydro-desmycosin (If) as well as its addition salts with inorganic or organic acids and intermediates (Ia-Ie) in the process of its preparation are new compounds and have hitherto not been described in the literature.

The first object of the present invention are novel derivatives of 10,11,12,13-tetrahydro-desmycosin represented by the formula I

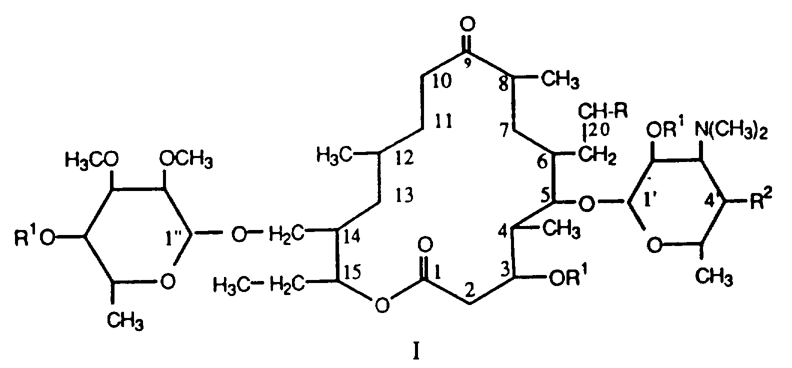

| | | | |
|---|---|---|---|
| Ia | R = (OC₂H₅)₂, | R¹ = H, | R = OH |
| Ib | R = (OC₂H₅)₂, | R¹ = Si(CH₃)₃, | R = OH |
| Ic | R = (OC₂H₅)₂, | R¹ = Si(CH₃)₃, | R = OSO₂CH₃ |
| Id | R = (OC₂H₅)₂, | R¹ = Si(CH₃)₃, | R = I |
| Ie | R = =O, | R¹ = H, | R = I |
| If | R = =O, | R¹ = H, | R = H |

and their pharmaceutically acceptable salts with organic or inorganic salts.

A further object of the present invention is a process for the preparation of derivatives of 10,11,12,13-tetrahydro-desmycosin by subjecting 10,11,12,13-tetrahydro-desmycosin of the formula II 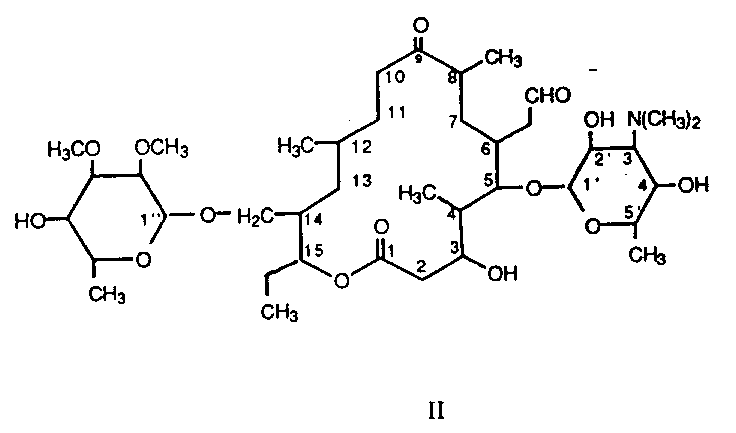 to acetalysation in ethanol in an equimolar quantity of *p*-toluenesulfonic acid. The end of the reaction is determined chromatographically (system B); upon a partial neutralisation by means of triethylamine, the solvent is evaporated to one quarter of its volume, a saturated solution of sodium hydrogen carbonate is added, and upon extraction with chloroform, 10,11,12,13-tetrahydro-desmycosin diethylacetal (Ia, R = (OC₂H₅)₂, R¹ = H, R = OH) is isolated. The compound Ia is subjected to selective silylation in position C-3,2' and 4'' with 6-8 equivalents of trimethylchlorosilane in the presence of a *tert.* organic amine such as e.g. pyridine, triethylamine or dimethylaniline, in an inert solvent such as e.g. dichloromethane, dichloroethane or chloroform, at a temperature of 0-5 °C. The termination of the reaction is determined chromatographically (system A); there is added a mixture of water and ice and upon extraction with chloroform at a pH of 8-9, there is isolated 3,2',4''- tri-O-trimethylsilyl-10,11,12,13-tetrahydro-desmycosin diethylacetal (Ib, R = (OC₂H₅)₂, R¹ = Si(CH₃)₃, R = OH). The crude product Ib is dissolved in pyridine and sulfonated in C-4' with 5-7 equivalents of the corresponding sulfochloride such as e.g. methanesulfonylchloride or benzyl-sulfonylchloride at a temperature of -5° to 15 °C. The termination of the reaction is determined chromatographically (system A), and upon addition of a mixture of water and ice and adjustment of the pH to 9-9.5, 4'-methanesulfonyl-3,2',4''-tri-O-trimethylsilyl-10,11,12,13-tetrahydro-desmycosin diethylacetal (Ic, R = (OC₂H₅)₂, R¹ = Si(CH₃)₃, R = OSO₂CH₃) is precipitated. The moist precipitate is dissolved in chloroform and treated with a saturated solution of sodium chloride. Upon drying the extract and the evaporation of the solvent at reduced pressure, the obtained product Ic is immediately converted into the 4'-iodo derivative by means of dissolving in a dry inert solvent and the addition of 4-6 equivalents of an alkali metal iodide, and stirred at an increased temperature, maximum at reflux temperature, up to the disappearance of the starting compound (system C). As inert solvents there may be used dimethoxyethane or methyl ethyl ketone, whereas the alkali metal iodides are e.g. KI, NaI or LiI. The reaction mixture is concentrated and, upon addition of water and ice, extracted with chloroform at a pH of 9-9.5. The extracts are worked up with an aqueous solution of sodium thiosulfate (10%) and upon evaporation at reduced pressure, 4'-deoxy-4'-iodo-3,2',4''-tri-O-trimethylsilyl-10,11,12,13-tetrahydro-desmycosin diethylacetal (Id, R = (OC₂H₅)₂, R¹ = Si(CH₃)₃, R = I) is isolated. The hydrolysis of the protecting group in the 4'-iodo derivative (Id) is performed in a 50% solution of acetonitrile and 0.2N HCl. The hydrolysis proceeds at room temperature and upon its termination (system A, system B) and adjustment of the pH at 9-9.5, there is isolated by means of extraction the 4'-deoxy-4'-iodo-10,11,12,13-tetrahydro-desmycosin (Ie, R = O, R¹ = H, R = I). The crude product is purified on silica gel in system A or C. The fractions of an Rf value of 0.85 (system A) are evaporated at reduced pressure. The obtained yellow amorphous product is dissolved in dry ethanol and subjected to deiodination in the presence of a catalytical quantity of 5-10% palladium-on-carbon (0.1-2.0 % w./w.), at a hydrogen pressure of 0.1-0.2 MPa at room temperature within 2-8 hours. The duration of the reduction is determined chromatographically (system A or C); upon the separation of the catalyst by means of filtration, a double volume of water is added to the product and it is extracted at a pH of 8-9. The extracts are washed with a saturated solution of sodium chloride. Upon evaporation at reduced pressure, there is obtained a white crystalline product 4'-deoxy-10,11,12,13-tetrahydro-desmycosin (If, R = O, R¹ = R = H).

The acid addition salts of 4'-deoxy-10,11,12,13-tetrahydro-desmycosin, which are another object of the present invention, are prepared by reacting 4'-deoxy-10,11,12,13-tetrahydro-desmycosin in a reaction-inert solvent with at least an equimolar quantity of an inorganic or organic acid such as e.g. hydrogen chloride, sulfuric acid, phosphoric acid, acetic acid, propionic acid, citric acid, tartaric acid. The isolation is achieved by precipitation with a non-solvent or most often by means of a lyophilisation procedure.

4'-Deoxy-10,11,12,13-tetrahydro-desmycosin and its addition salts exhibit a potent antibacterial activity. The results of *in vitro* investigations are represented in Tables I and II in comparison with the parent antibiotic tylosin and its 10,11,12,13-tetrahydro derivatives.

According to the obtained results 4'-deoxy-10,11,12,13-tetrahydro-desmycosin may be used as antibiotic in the treatment of various infectious diseases, by means of administration in conventional pharmaceutical formulations.

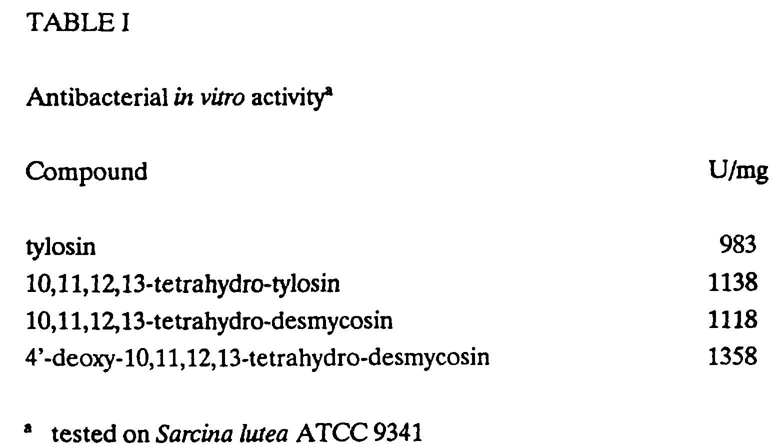

The synthesis of the novel derivatives of 10,11,12,13-tetrahydro-desmycosin was followed by TLC (thin layer chromatography) on silica gel in the following solvent systems:

| | |
|---|---|
| system A:methylene chloride:methanol:conc.NH₄OH | 90:9:1.5 |
| system B:ethyl acetate:methanol:conc.NH₄OH | 85:10:5 |
| systeme C:benzene:acetone | 4:1 |

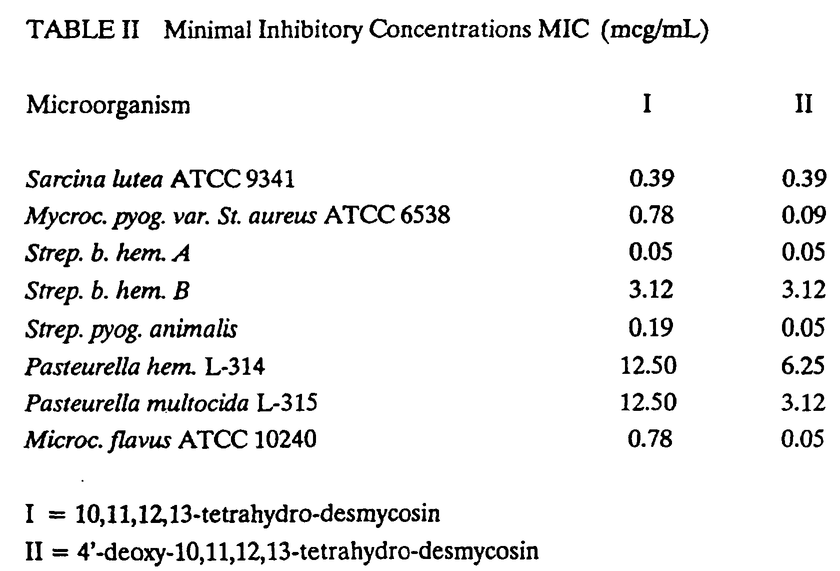

The invention is illustrated by the following Examples which are not to be construed as limiting its scope.

### EXAMPLE 1

### 10,11.12, 13-Tetrahydro-desmycosin diethylacetal (Ia)

10,11,12,13-tetrahydro-desmycosin (50 g; 64.4 mmole) was dissolved in 500 mL of dry ethanol and p-toluenesulfonic acid monohydrate (12.5 g; 65 mmole) was added thereto. Upon stirring for two hours at room temperature, 6 mL of triethylamine were added, the ethanol was evaporated at reduced pressure to one quarter of the volume, then there were added 700 mL of a saturated solution of sodium hydrogen carbonate and it was extracted with chloroform (2 x 100 mL).

The extracts were dried over potassium carbonate and evaporated at reduced pressure into a dry residue.

There were obtained 50.9 g (93%), Rf (A) 0.30, Rf (B) 0.50.
- IR(KBr)cm⁻¹: 3480, 2970, 1720, 1460, 1380, 1265, 1170, 1085, 1060, 1010, 960.
- ¹H-NMR(CDCl₃)ppm: 3.61(3H,3"OCH₃),3.56(2H,20-OCH₂-), 3.50(3H,2"OCH3),3.45(2H,20-OCH₂-), 2.49(6H,N(CH₃)₂).
- ¹³C-NMR(CDCl₃)ppm: 215.07(C-9), 172.52(C-1), 105.31(C-1') 102.31(C-20), 100.62(C-1"), 61.80(20-OCH₂-), 61.63(3"-OCH₃), 60.62(20-O-CH₂-), 59.31(2"OCH₃)

### EXAMPLE 2

### 3,2',4"-Tri-O-trimethylsilyl-10,11,12, 13-tetrahydro-desmycosin diethylacetal (Ib)

10,11,12,13-Tetrahydro-desmycosin diethylacetal (Ia), (10 g; 11.7 mmole) was dissolved in 200 mL of dry methylene chloride and 7.8 mL (96.6 mmole) of dry pyridine. The solution was cooled to 0 °C and then there were added dropwise 11 mL (87 mmole) of trimethylchlorosilane. Upon stirring for 2 hours at 5 °C, it was poured into 400 mL of an ice-water mixture, adjusted to a pH of 9 by the addition of conc. NH₄OH and extracted with chloroform (2x100 mL). The extracts were washed with a saturated solution of sodium chloride, dried over potassium carbonate and evaporated at reduced pressure.

There were obtained 12.0 g (96%), Rf (A) 0.75.
- IR(KBr)cm⁻¹: 2970, 1730, 1460, 1380, 1265, 1255, 1170, 1100, 1085, 1060, 1010, 970, 885, 842, 755
- ¹H-NMR(CDCl₃)ppm: 3.59(5H,3"OCH₃,20-OCH₂-), 3.51(5H,2"OCH₃, 20-OCH₂-), 2.52(6H,N(CH₃)₂)0.17(27H,3xSi(CH₃)₃)

### EXAMPLE 3

### 4'-Methanesulfonyl-3,2',4"-tri-O-trimethylsilyl-10,11,12,13-tetrahydro-desmycosin diethylacetal (Ic)

The product Ib (12 g; 11.25 mmole) was dissolved in 100 mL of pyridine. Into the cooled solution (10 °C) there were added 5.2 mL (67 mmole) of methanesulfo-chloride and it was kept stirring under cooling for 4 hours. The reaction solution was poured into 1500 mL of ice-water and the pH was adjusted to 9 by the addition of conc. NH₄OH. After 30 minutes the precipitate was separated by filtration and immediately dissolved in 100 mL of chloroform. The solution was thoroughly washed with a saturated solution of sodium chloride, dried over MgSO₄ and evaporated into a dry residue.
There were obtained 12.1 g (94%), Rf (A) 0.90.
- IR(KBr)cm⁻¹: 2970, 1730, 1460, 1380, 1360, 1265, 1255, 1170, 1100, 1085, 1060, 965, 885, 842, 755.
- ¹H-NMR(CDCl₃)ppm: 3.59(5H,3"OCH₃,20-OCH₂-), 3.51(5H,2"OCH₃, 20-OCH₂-), 3.15(3H,SO₂-CH₃),2.54 and 2.49(6H,N(CH₃)₂),0.16(27H,3xSi(CH₃)₃)

### EXAMPLE 4

### 4'-Deoxy-4'-iodo-3,2',4"-tri-O-trimethylsilyl-10,11,12,13-tetrahydro-desmycosin diethylacetal (Id)

The compound Ic (12 g; 10.5 mmole) was dissolved in 120 mL of methyl ethyl ketone, then there were added 7.8 g (52 mmole) of sodium iodide and it was heated under a very mild reflux for two hours. The solvent was evaporated at reduced pressure to one tenth of its volume, whereupon there were added 100 mL of chloroform and 200 mL of water, it was adjusted with alkali to a pH of 9 and the layers were separated. The organic compound was washed with a 10% solution of sodium thiosulfate (2x100 mL). The evaporation under reduced pressure yielded 11.22 g (90.9%) of the yellow title product. Rf (A) 0.95, Rf (C) 0.85.
- IR(KBr)cm⁻¹: 2970, 1725, 1460, 1380, 1265, 1255, 1170, 1100, 1085, 1060, 965, 885, 842, 755.
- ¹H-NMR(CDCl₃)ppm: 3.59(5H,3"OCH₃,20-OCH₂-), 3.50(5H,2"OCH₃, 20-OCH₂-), 2.54 and 2.49(6H,N(CH₃)₂)0.16(27H,3xSi(CH₃)₃).

### EXAMPLE 5

### 4'-Deoxy-4'-iodo-10,11,12,13-tetrahydro-desmycosin (Ie)

The compound Id (11 g; 9.3 mmole) was dissolved in 110 mL of a mixture of acetonitrile and 110 mL of 0.2N HCl and stirred at room temperature for two hours. Upon addition of solid sodium hydrogen carbonate up to a pH of 9, it was extracted with chloroform (2x60 mL). The extracts were washed with a saturated solution of sodium hydrogen carbonate and evaporated at reduced pressure. There were obtained 7.3 g (88.6%) of the crude product, which was dissolved in a small quantity of methylene chloride and purified by chromatography on a silica gel column (Silica gel 60, Merck Co., 70-230 mesh) in solvent system A. The evaporation of the fractions of a Rf (A) 0.85) yielded 3.44 g (55%) of a chromatographically pure product, Rf (C) 0.30.
- IR(KBr)cm⁻¹: 3480, 2970, 1720, 1460, 1380, 1260, 1170, 1085, 1060, 1010, 960.
- ¹H-NMR(CDCl₃)ppm: 9.67(1H,20-CHO),3.61(3H,3"OCH₃), 2.49(3H,2"OCH₃),2.58 i 2.56(6H,N(CH₃)₂).
- ¹³C-NMR(CDCl₃)ppm: 214.68(C-9), 202.60(C-20), 172.35(C-1), 61.63(3"-OCH₃), 59.43(2"OCH₃), 30.70(C-4').

### EXAMPLE 6

### 4'-Deoxy-10,11,12,13-tetrahydro-desmycosin (If)

### Process A

The compound Ie (1 g; 1.1 mmole) was dissolved in 50 mL of dry ethanol. Upon addition of 0.2 g of 10% palladium-on-carbon, it was hydrogenated for 2 hours at room temperature at a hydrogen pressure of 0.2 MPa. The catalyst was separated by filtration, the ethanol was evaporated at reduced pressure into an oily product, 100 mL of water were added, whereupon it was extracted with chloroform at a pH of 8.5. The extracts were dried over potassium carbonate and evaporated at reduced pressure into a white crystalline product.

There were obtained 0.73 g (85.9%), Rf (A) 0.35, M⁺ 759
- IR(KBr)cm⁻¹: 3480, 2970, 1720, 1460, 1380, 1260, 1170, 1085, 1060, 960.
- ¹H-NMR(CDCl₃)ppm: 9.67(1H,20-CHO),3.62(3H,3"OCH₃), 3.50(3H,2"OCH₃),2.26(6H,N(CH₃)₂),1.25(1H,4'),1.18(1H,4').
- ¹³C-NMR(CDCl₃)ppm: 214.79(C-9), 202.99(C-20), 172.47(C-1), 61.74(3"-OCH₃), 59.43(2"OCH₃), 28.27(C-4').

### Process B

The compound Ie (1 g; 1.1 mmole) was dissolved in 50 mL of dry ethanol, 0.1 g of 5% palladium-on-carbon were added and it was hydrogenated for 8 hours at room temperature at a hydrogen pressure of 0.1 MPa. After the completed hydrogenation the catalyst was separated by filtration, whereupon an identical isolation as in process A was performed. There was obtained a product with the same characteristics as in process A.

## Claims

1. Derivatives of 10,11,12,13-tetrahydro-desmycosin of the formula (I) 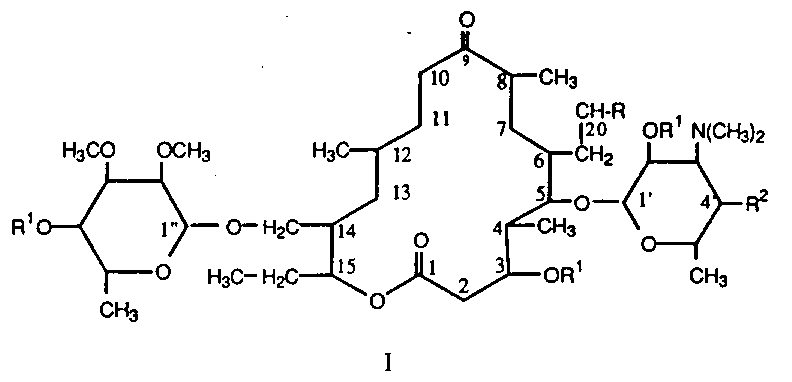 wherein
*R* *represents O when R¹ stands for H and R stands for H or I; or*
*R* *represents (OC₂H₅)₂ when R¹ stands for Si(CH₃*)*₃ and R stands for OH, OSO₂CH₃, I or H; or when R¹ stands for H and R stands for H or OH.*
and their pharmaceutically acceptable salts with inorganic or organic acids.

2. 10,11,12,13-tetrahydro-desmycosin diethylacetal.

3. 3,2',4"-Tri-O-trimethylsilyl-10,11,12,13-tetrahydro-desmycosin diethylacetal.

4. 4'-Methanesulfonyl-3,2',4"-tri-O-trimethylsilyl-10,11,12,13-tetrahydro-desmycosin diethylacetal.

5. 4'-Deoxy-4'-iodo-3,2',4"-tri-O-trimethylsilyl-10,11,12,13-tetrahydro-desmycosin diethylacetal.

6. 4'-Deoxy-4'-iodo-10,11,12,13-tetrahydro-desmycosin.

7. 4'-Deoxy-10,11,12,13-tetrahydro-desmycosin.

8. A process for the preparation of derivatives of 10, 11,12,1 3-tetrahydro-desmycosin of the formula I 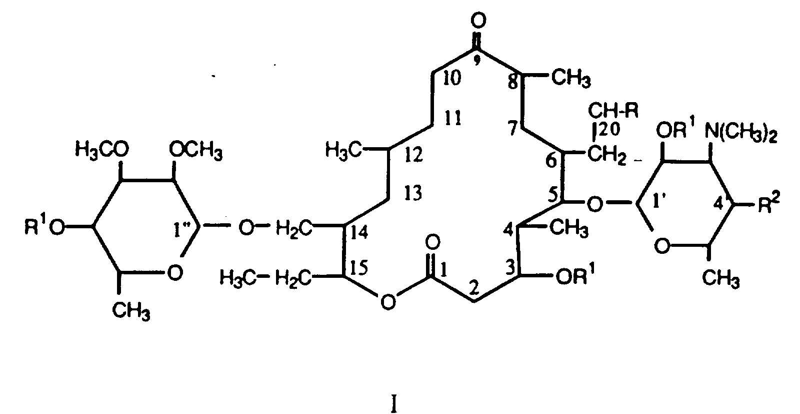 wherein
*R* *represents O when R¹ stands for H and R stands for H or I; or*
*R* *represents (OC₂H₅)₂ when R¹ stands for Si(CH₃)₃ and R stands for OH*, *OSO₂CH*₃, *I or H; or when R¹ stands for H and R* *stands for H or OH.*
characterised in that 10,11,12,13-tetrahydro-desmycosin of the formula II 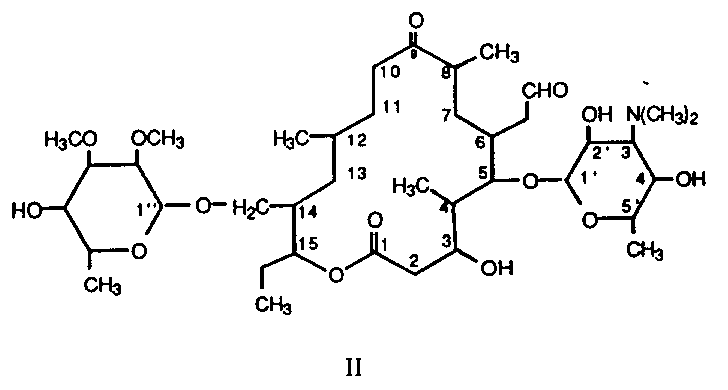 is subjected to acetalysation in ethanol in an equimolar quantity of *p*-toluenesulfonic acid at room temperature for 2 hours, whereupon triethylamine is added, ethanol is evaporated to one quarter of its volume, there is added a saturated solution of sodium hydrogen carbonate and, upon extraction with chloroform, there is isolated 10,11,12,13-tetrahydro-desmycosin diethylacetal (Ia), where R means (OC₂H₅)₂, R¹ means H, R means OH; said compound is then subjected to selective silylation in positions C-3,2' and 4" with 6-8 equivalents of trimethylchlorosilane in the presence of a *tert.* organic amine such as pyridine, triethylamine or dimethylaniline, in an inert solvent such as e.g. dichloromethane, dichloroethane or chloroform, at a temperature of 0-5 °C for 2 hours, whereupon there is added a mixture of water and ice and it is extracted with chloroform at a pH of 8-9 to isolate 3,2',4"-tri-O-trimethylsilyl-10,11,12,13-tetrahydro-desmycosin diethylacetal (Ib), wherein R means (OC₂H₅)₂, R¹ means Si(CH₃)₃, and R means OH; said product is dissolved in pyridine and subjected to sulfonation with 5-7 equivalents of methanesulfonylchloride at a temperature of -5° to 15 °C for 4 hours, the reaction solution is poured into a mixture of water and ice, adjusted with alkali to a pH of 9-9.5, the obtained precipitate is separated by filtration and then dissolved in chloroform, treated with a saturated solution of sodium chloride and upon drying and evaporation at reduced pressure there is obtained 4'-methane-sulfonyl-3,2',4"-tri-O-trimethylsilyl-10,11,12,13-tetrahydro-desmycosin diethylacetal (Ic), wherein R means a (OC₂H₅)₂ group, R¹ means a Si(CH₃)₃ group, and R means a OSO₂CH₃ group; said product is dissolved in a dry inert solvent such as dimethoxyethane, methyl ethyl ketone, there are added 4-6 equivalents of an alkali metal iodide, such as KI, NaI or LiI, and it is stirred at a mild reflux temperature for 2 hours, whereupon the solvent is evaporated at reduced pressure to one tenth of its volume, and upon addition of water and ice the pH is adjusted to 9-9.5 and it is extracted with chloroform, the extracts are worked up with a 10% solution of sodium thiosulfate and upon evaporation at reduced pressure there is isolated 4'-deoxy-4'-iodo-3,2',4"-tri-O-trimethylsilyl-10,11,12,13-tetrahydro-desmycosin diethylacetal (Id), wherein R means (OC₂H₅)₂, R¹ means Si(CH₃)₃, and R means iodine; said compound is subjected to a hydrolysis of the protecting groups in a 50% solution of acetonitrile and 0.2N HCI, at room temperature for 2 hours, then there is added sodium hydrogen carbonate up to a pH of 9, upon extraction with chloroform the crude product is isolated, which, upon purification on a silica gel column, yields 4'-deoxy-4'-iodo-10,11,12,13-tetrahydro-desmycosin (Ie), wherein R means O, R¹ means H, and R means iodine; said product is subjected to deiodination in dry ethanol in the presence of a catalytical quantity of 5-10% palladium-on-carbon at a hydrogen pressure of 0.1-0.2 MPa at room temperature within 2-8 hours; upon the separation of the catalyst by means of filtration there is added a double volume of water and it is extracted at a pH of 8-9, the extracts are washed with a saturated solution of sodium chloride, and upon evaporation at reduced pressure there is obtained a white crystalline product 4'-deoxy-10,11,12,13- tetrahydro-desmycosin (If), wherein R means O, and R¹ and R are identical and mean hydrogen; and the product is optionally converted into a pharmaceutically acceptable addition salt by being subjected to reaction with at least one equivalent of a corresponding organic or inorganic acid.

9. The use of the compound as claimed in claim 7, in obtaining pharmaceutical preparations of antibacterial activity, in combination with a pharmaceutically acceptable carrier.

## Patentansprüche

1. Derivate von 10,11,12,13-Tetrahydro-desmycosin der Formel (I) 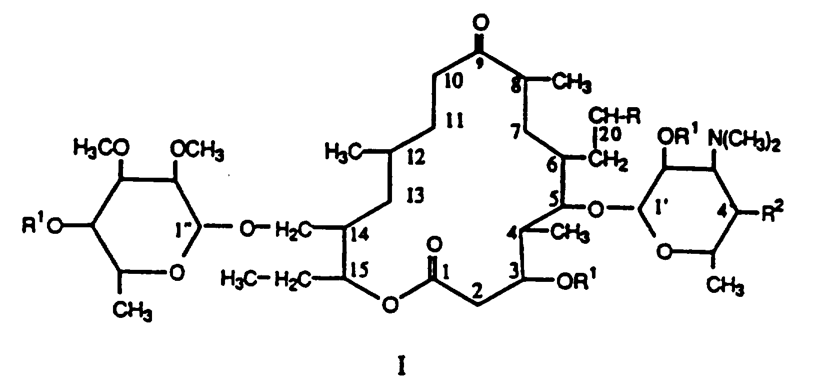 worin
R = O, falls R¹ für H und R für H oder J stehen; oder
R = (OC₂H₅)₂, falls R¹ für Si(CH₃)₃ und R für OH, OSO₂CH₃, J oder H stehen; oder falls R¹ für H und R für H oder OH stehen,
und deren pharmazeutisch annehmbare Additionsalze mit anorganischen oder organischen Säuren.

2. 10,11,12,13-Tetrahydro-desmycosin-diäthylacetal.

3. 3,2,4".Tri-O-trimethylsilyl-10,11,12,13-tetrahydro-desmycosin-diäthylacetal.

4. 4'-Methansulfony1-3,2',4''-tri-O-trimethylsilyl-10,11,12,13-tetrahydro-desmycosin-diäthylacetal.

5. 4'-Deoxy-4'-jodo-3,2',4"-tri-O-trimethylsilyl-10,11,12,13-tetrahydro-desmycosindiäthylacetal.

6. 4'-Deoxy-4'-jodo-10,11,12,13-tetrahydro-desmycosin.

7. 4'-Deoxy-10,11,12,13-tetrahydro-desmycosin.

8. Verfahren zur Herstellung von 10,11,12,13-Tetrahydro-desmycosin-Derivaten der Formel (I) 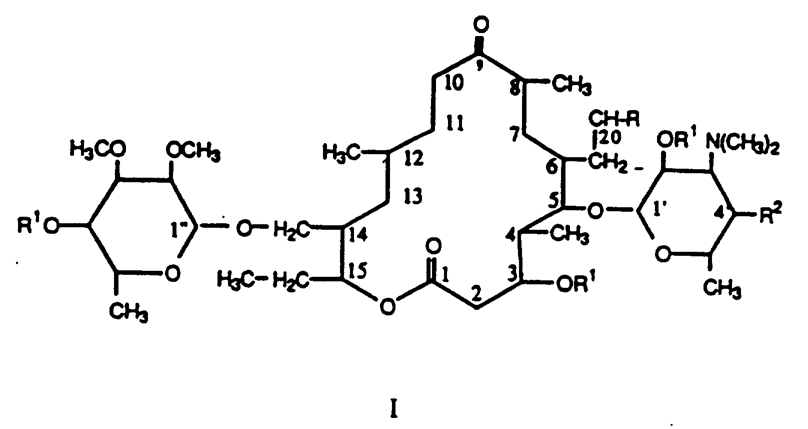 worin
R = O, falls R¹ für H und R für H oder J stehen; oder
R = (OC₂H₅)₂, falls R¹ für Si(CH₃)₃ und R für OH, OSO₂CH₃, J oder H stehen; oder falls R¹ für H und R für H oder OH stehen,
dadurch gekennzeichnet, dass 10,11,12,13-Tetrahydro-desmycosin der Formel (II) 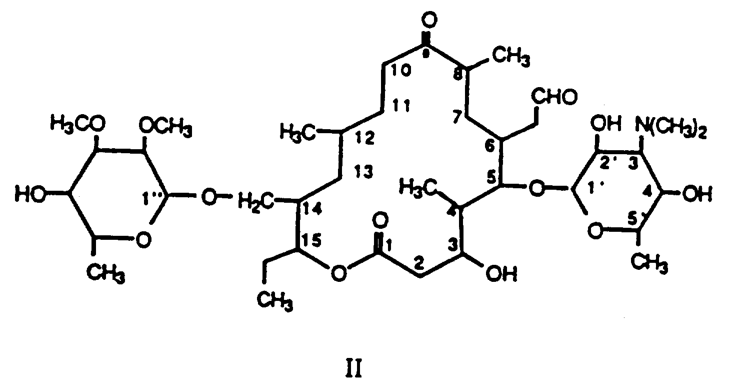 einer Acetalysierung in Äthanol in einer äquimolaren Menge von *p*-Toluolsulfonsäure bei Raumtemperatur während 2 Stunden unterworfen wird, dann Triäthylamin zugegeben wird und Äthanol auf ein Viertel seines Volumens eingedampft wird, anschliessend eine gesättigte Lösung von Natriumhydrogencarbonat zugegeben wird und nach einer Extraktion mit Chloroform 10,11,12,13-Tetrahydro-desmycosin-diäthylacetal (Ia), worin R für (OC₂H₅)₂, R¹ für H und R für OH stehen, erhalten wird, das dann einer selektiven Silylierung an C-3,2'- und 4"-Stellungen mit 6-8 Äquivalenten von Trimethylchlorosilan in Anwesenheit von einem *tert*.-organischen Amin wie z.B. Pyridin, Triäthylamin oder Dimethylanilin, in einem inerten Lösungsmittel wie z.B. Dichlormethan, Dichloräthan oder Chloroform bei einer Temperatur von 0 - 5°C während 2 Stunden unterworfen wird, wonach ein Gemisch von Wasser und Eis zugegeben wird und mit Chloroform bei einem pH von 8-9 extrahiert wird, um 3,2',4"-Tri-O-trimethyl-silyl-10,11,12,13-tetrahydro-desmycosin-diäthylacetal (Ib). worin R für (OC₂H₅)₂, R¹ für Si(CH)₃ und R für OH stehen, zu erhalten, das in Pyridin gelöst und mit 5-7 Äquivalenten von Methansulfonylchlorid bei einer Temperatur von -5°C bis 15°C während 4 Stunden sulfoniert wird, anschliessend die Reaktionsmischung in ein Gemisch von Wasser und Eis eingegossen wird und das pH mittels Alkali auf 9-9.5 eingestellt wird, das erhaltene Präzipitat durch Filtration getrennt und anschliessend in Chloroform gelöst und dann mit einer gesättigten Natriumchloridlösung behandelt wird, wobei nach Trocknung und Verdampfung bei vermindertem Druck 4'-Methansulfonyl-3,2',4"-tri-O-trimethylsilyl-10,11,12,13-tetrahydro-desmycosindiäthylacetal (Ic), worin R für (OC₂H₅)₂, R¹ für Si(CH)₃ und R für OSO₂CH₃ stehen, erhalten wird, das in einem inerten trockenen Lösungsmittel wie z.B. Dimethoxyäthan, Methyläthylketon gelöst wird und zu dem 4-6 Äquivalenten von Alkalimetalljodid wie z.B. KJ, NaJ oder LiJ zugegeben werden, wonach bei einer milden Rückflusstemperatur während 2 Stunden gerührt wird, worauf das Lösungsmittel bei vermindertem Druck auf ein Zehntel seines Volumens eingedampft wird und nach der Zugabe von Wasser und Eis das pH auf 9-9,5 eingestellt wird, wonach mit Chloroform extrahiert wird und die Extrakte mit einer 10%-en Lösung von Natriumthiosulfat behandelt werden, wobei nach Verdampfung bei vermindertem Druck 4'-Deoxy-4'-jodo-3,2',4"-tri-O-trimethylsilyl-10,11,12,13-tetrahydro-desmycosin-diäthylacetal (Id), worin R für (OC₂H₅)₂, R¹ für Si(CH₃)₃ und R für Jod stehen, erhalten wird, das einer Hydrolyse der schützenden Gruppen in einer 50%-en Lösung von Acetonitril und 0,2 N HCl bei Raumtemperatur innerhalb von 2 Stunden unterworfen wird, wonach Natriumhydrogencarbonat bis zu einem pH-Wert von 9 zugegeben wird und nach der Extraktion mit Chloroform das Rohprodukt isoliert wird, wobei nach Reinigung an einer Silikagelsäule 4'-Deoxy-4'-jodo-10,11,12,13-tetrahydro-desmycosin (Ie), worin R für O, R¹ für H und R für Jod stehen, erhalten wird, das einer Dejodierung in trockenem Äthanol in Gegenwart einer katalytischen Menge von 5-10% Palladium-auf-Kohle bei einem Wasserstoffdruck von 0,1-0,2 MPa bei Raumtemperatur innerhalb von 2-8 Stunden unterworfen wird, nach der Trennung des Katalysators mittels Filtrierung ein doppeltes Volumen von Wasser zugefügt wird und anschliessend bei einem pH von 8-9 extrahiert wird, die Extrakte mit einer gesättigten Natriumchloridlösung gewaschen werden und anschliessend nach der Verdampfung bei vermindertem Druck ein weisses kristallinisches Produkt 4'-Deoxy-10,11,12,13-tetrahydro-desmycosin (If), worin R für O steht und R¹ und R gleich sind und für H stehen, erhalten wird, das gegebenenfalls durch Umsetzung mit wenigstens einem Äquivalenten einer entsprechenden organischen oder anorganischen Säure in ein pharmazeutisch annehmbares Additionsalz umgesetzt wird.

9. Verwendung der Verbindung nach Anspruch 7 zur Herstellung von pharmazeutischen Zubereitungen mit antibakterieller Wirkung in einer Kombination mit einem pharmazeutisch annehmbaren Träger.

## Revendications

1. Dérivés de la 10,11,12,13-tétrahydrodesmycosine de la formule (I) 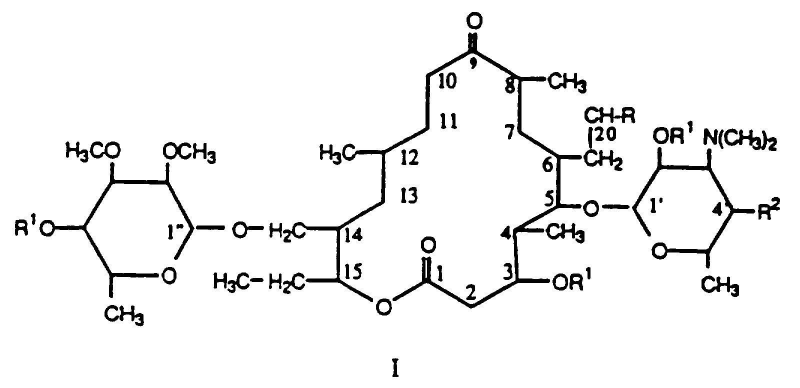 dans laquelle
R représente O lorsque R¹ représente H et R représente H ou I, ou bien
R représente (OC₂H₅)₂ lorsque R¹ représente Si(CH₃)₃ et Rreprésente OH, OSO₂CH₃, I ou H, ou lorsque R¹ représente H et R représente H ou OH
ainsi que leurs sels pharmaceutiquement acceptables avec des acides inorganiques ou organiques.

2. 10,11,12,13-Tétrahydrodesmycosinediéthylacétal.

3. 3,2',4"-Tri-O-triméthylsilyl-10,11,12,13-tétra-hydrodesmycosinediéthylacétal.

4. 4'-Méthanesulfonyl-3,2',4"-tri-O-triméthylsilyl-10,11,12,13-tétrahydrodesmycosindiéthylacétal.

5. 4'-Désoxy-4'-iodo-3,2',4"-tri-O-triméthylsilyl-10,11,12,13-tétrahydrodesmycosinediéthylacétal.

6. 4'-Désoxy-4'-iodo-10,11,12,13-tétrahydrodesmycosine.

7. 4'-Désoxy-10,11,12,13-tétrahydrodesmycosine.

8. Procédé de préparation de la 10,11,12,13-tétrahydrodesmycosine de la formule (I) 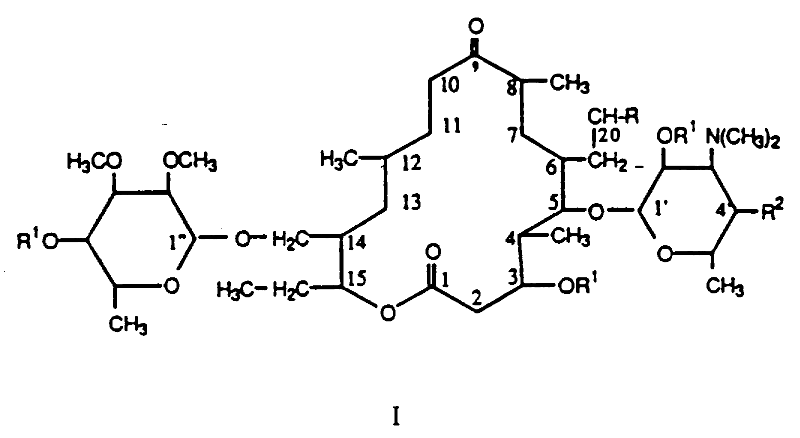 dans laquelle
R représente O lorsque R¹ représente H et R représente H ou I, ou bien
R représente (OC₂H₅)₂ lorsque R¹ représente Si(CH₃)₃ et Rreprésente OH, OSO₂CH₃, I ou H, ou lorsque R¹ représente H et R représente H ou OH,
caractérisé en ce que l'on soumet la 10,11,12,13-tétrahydrodesmycosine de la formule II 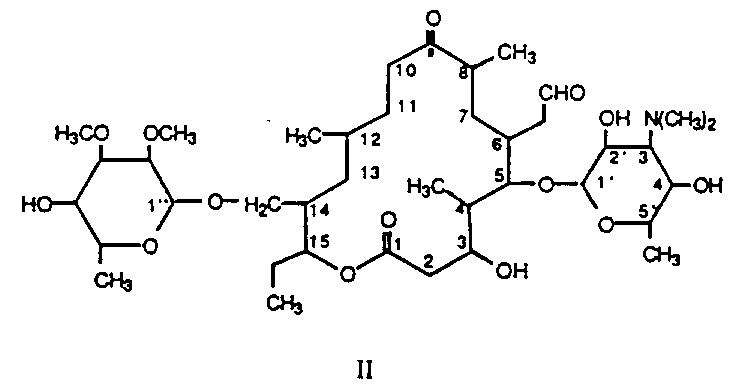 à une acétalisation dans l'éthanol dans une quantité équimolaire d'acide p-toluènesulfonique, à la température ambiante, pendant 2 heures, puis on ajoute de la triéthylamine, on évapore l'éthanol jusqu'au quart de son volume, on ajoute une solution saturée de carbonate hydrogéné de sodium et, par extraction avec du chloroforme, on isole le 10,11,12,13-tétrahydrodesmycosinediéthylacétal (Ia), où R représente (OC₂H₅)₂, R¹ représente H, R représente OH, on soumet ensuite ledit composé à une silylation sélective en positions C-3,2' et 4" avec 6-8 équivalents de triméthylchlorosilane, en présence d'une amine organique tertiaire, comme la pyridine, la triéthylamine ou la diméthylaniline, dans un solvant inerte, comme, par exemple, le dichlorométhane, le dichloréthane ou le chloroforme, à une température de O à 5°c pendant 2 heures, puis on ajoute un mélange d'eau et de glace et on l'extrait avec du chloroforme à un pH de 8-9 pour isoler le 3,2',4"-tri-O-triméthylsilyl-10,11,12, 13-tétrahydrodesmycosinediéthylacétal (Ib), où R représente (OC₂H₅)₂, R¹ représente (Si(CH₃)₃ et R représente OH, on dissout le produit dans de la pyridine et on soumet la solution à sulfonation avec 5 à 7 équivalents de chlorure de méthanesulfonyle à une température de -5 à 15°c pendant 4 heures, on verse la solution réactionnelle dans un mélange d'eau et de glace, on ajuste le pH avec un alcali à 9-9,5, on sépare le précipité obtenu par filtration et on le dissout ensuite dans du chloroforme, on traite la solution par une solution saturée de chlorure de sodium et, par séchage et évaporation sous pression réduite, on obtient le 4'-méthanesulfonyl-3,2',4"-tri-O-triméthylsilyl-10,11,12,13-tétra-hydrodesmycosinediéthylacétal (Ic), où R représente un groupe (OC₂H₅)₂, R¹ représente un groupe Si(CH₃)₃ et R représente un groupe OSO₂CH₃, on dissout le produit en question dans un solvant inerte et sec, comme le diméthoxyéthane, la méthyléthylcétone, on ajoute 4 à 6 équivalents d'un iodure de métal alcalin, tel que KI, NaI ou LiI et on agite le tout à une température de reflux modérée pendant 2 heures, puis on évapore le solvant sous pression réduite jusqu'à un dixième de son volume, et, par addition d'eau et de glace, on ajuste le pH à 9-9,5 et on procède à une extraction avec du chloroforme, on traite les extraits par une solution à 10% de thiosulfate de sodium et, par évaporation sous pression réduite, on isole le 4'-désoxy-4'-iodo-3,2',4"-tri-O-triméthylsilyl-10,11,12,13-tétrahydrodesmycosinediéthyl-acétal (Id), où R représente (OC₂H₅)₂, R¹ représente Si(CH₃)₃ et R représente l'iode, on soumet ledit composé à une hydrolyse des radicaux protecteurs dans une solution à 50% d'acétonitrile et d'HCl 0,2N, à la température ambiante et pendant 2 heures, puis on ajoute de l'hydrogénocarbonate de sodium jusqu'à un pH de 9, on isole le produit brut par extraction au chloroforme, lequel produit brut, par purification sur une colonne de gel de silice, donne la 4'-désoxy-4'-iodo-10,11,12,13-tétrahydrodesmycosine (Ie), où R représente O, R¹ représente H et R représente l'iode, on soumet ledit produit à une désiodation dans de l'éthanol sec en présence d'une quantité catalytique de palladium à 5-10% sur carbone, sous une pression d'hydrogène de 0,1-0,2 MPa à la température ambiante en 2 à 8 heures, après la séparation du catalyseur par filtration, on ajoute un double volume d'eau et on procède à une extraction au pH de 8-9, on lave les extraits avec une solution saturée de chlorure de sodium et, par évaporation sous pression réduite, on obtient un produit cristallin blanc constitué de 4'-désoxy-10,11,12,13-tétrahydrodesmycosine (If), où R représente O et R¹ et R sont identiques et représentent de l'hydrogène et on convertit éventuellement le produit en un sel d'addition pharmaceutiquement acceptable en le soumettant à réaction avec au moins un équivalent de l'acide organique ou inorganique correspondant.

9. Utilisation du composé suivant la revendication 7 en vue de l'obtention de préparations pharmaceutiques à activité antibactérienne, en combinaison avec un véhicule ou excipient pharmaceutiquement acceptable.
